# EUROPEAN PATENT APPLICATION

(11) **EP 4 414 446 A2**
(43) Date of publication of application: **14.08.2024**
(21) Application number: 24154402.2
(22) Date of filing: 29.01.2024
(51) Int. Cl.: C12M 3/00, A61L 27/22, A61L 27/38, A61L 27/56, C07K 14/78, C12M 1/00, C12M 1/12, C12N 5/00

(54) **POROUS STRUCTURE AND METHOD FOR PRODUCING POROUS STRUCTURE**

(30) Priority: 09.02.2023 JP 2023018664; 15.12.2023 JP 2023211964
(71) Applicant: Ricoh Company, Ltd., Tokyo 143-8555 (JP)
(72) Inventor: KUBO, Chihiro, Tokyo, 143-8555 (JP); SATOH, Naoki, Tokyo, 143-8555 (JP); YAGINUMA, Hidekazu, Tokyo, 143-8555 (JP)
(74) Representative: Marks & Clerk LLP

(57) **Abstract**

The present invention provides a porous carrier having high porosity and excellent in shape retention, and provides a method for producing the porous carrier. A porous structure is formed by: preparing a liquid mixture comprising at least a first polypeptide and a second polypeptide; and adding transglutaminase to the liquid mixture, and allowing the resulting mixture to react to form a gel.

## Description

### BACKGROUND OF THE INVENTION

### Field of the Invention

The present invention relates to a porous carrier mainly for use in cell culture and to a method for producing the porous carrier.

### Description of the Related Art

In the fields of regenerative medicine and drug-discovery screening, recent interest has increasingly focused on tissue engineering, accompanied by increasing need for three-dimensional cell culture. A three-dimensional cell-culture material usually has a problem in that neither oxygen nor nutrients reach the central part of the material, causing cells in the central part to be prone to die. A known method for solving this problem is a method in which a porous structure is used as a cell culture carrier. Non-Patent Literature 1 discloses a method in which gelatin is crosslinked using transglutaminase, thus formed into gel, and lyophilized to produce a porous structure. Patent Literature 1 discloses a method in which gelatin is chemically crosslinked using carbodiimide or the like or physically crosslinked by UV curing or the like to be formed into a gel, which is lyophilized to produce a porous structure.

### Citation List

### Patent Literature

[Patent Literature 1] Japanese Unexamined Patent Application Publication No. 2016-21984

### Non-Patent Literature

[Non-Patent Literature 1] Long et al., Preparation and characteristics of gelatin sponges crosslinked by microbial transglutaminase, PeerJ 5: e3665 (2017)

### SUMMARY OF THE INVENTION

As described in Patent Literature 1 and Non-Patent Literature, porous carriers for cell culture have been produced. However, the technologies according to Patent Literature 1 and Non-Patent Literature 1 have a problem in that making the porosity sufficiently higher causes the mechanical strength of the resulting porous structure to be prone to be lower.

The present disclosure has an object to provide a porous carrier having high porosity and excellent in shape retention, and provide a method for producing the porous carrier.

The present disclosure provides the following.

### <Porous Structure>

A porous structure comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the porous structure.

### <Method for Producing Porous Structure>

A method for producing a porous structure, comprising: preparing a liquid mixture comprising at least a first polypeptide and a second polypeptide; adding transglutaminase to the liquid mixture and allowing the resulting mixture to react to form a gel; and
forming the porous structure from the gel.

The present disclosure can provide a porous carrier having high porosity and excellent in shape retention, and provide a method for producing the porous carrier.

### BRIEF DESCRIPTION OF THE DRAWINGS

FIG. 1 is a schematic cross-sectional view illustrating an example of a porous structure in a first embodiment;
FIG. 2 is a schematic diagram illustrating an example of the molecular architecture of the porous structure in the first embodiment;
FIG. 3 is a schematic diagram illustrating an isopeptide bond in the molecular architecture of the porous structure in the first embodiment;
FIG. 4 is a schematic diagram illustrating an example of the molecular architecture of a conventional porous structure;
FIG. 5 presents images observed under a microscope, and illustrates an example of the porous structure in the first embodiment; FIG. 5A is an image of the whole porous structure, and FIG. 5B is an enlarged image of FIG. 5A;
FIG. 6 is related to an example of the porous structure in the first embodiment, and is an image of a frozen section observed under a phase-contrast microscope, in which the frozen section was obtained by immersing a section of the porous structure in PBS (-), and then freezing the section;
FIG. 7 presents an image of NIH/3T3 cells cultured using the hydrogel structure of the fourth embodiment, observed with a phase contrast microscope.
FIG. 8 presents an image of HepG2 cells cultured using the hydrogel structure of the fourth embodiment, observed with a phase contrast microscope.
FIG. 9 presents an image of PC12 cells cultured using the hydrogel structure of the fourth embodiment, observed with a phase contrast microscope.
FIG. 10 presents observational images of a porous structure produced in a 96-well plate in Example 10; FIG. 10A is a photographed image of the front face of the 96-well plate, and FIG. 10B is a photographed image of the back face of the 96-well plate;
FIG. 11 presents images observed under a phase-contrast microscope, and illustrates a porous structure in Example 1-1; FIG. 11A is a bright-field image, and FIG. 11B is a fluorescent image;
FIG. 12 presents images observed under a phase-contrast microscope, and illustrates a porous structure in Comparative Example 1-1; FIG. 12A is a bright-field image, and FIG. 12B is a fluorescent image;
FIG. 13 presents images observed under a phase-contrast microscope, and illustrates a porous structure in Example 2-1; FIG. 13A is a bright-field image, and FIG. 13B is a fluorescent image;
FIG. 14 presents images observed under a phase-contrast microscope, and illustrates a porous structure in Example 2-2; FIG. 14A is a bright-field image, and FIG. 14B is a fluorescent image;
FIG. 15 presents images observed under a phase-contrast microscope, and illustrates a porous structure in Comparative Example 2-1; FIG. 15A is a bright-field image, and FIG. 15B is a fluorescent image;
FIG. 16 presents images observed under a phase-contrast microscope, and illustrates a porous structure in Example 3-1; FIG. 16A is a bright-field image, and FIG. 16B is a fluorescent image;
FIG. 17 presents images observed under a phase-contrast microscope, and illustrates a porous structure in Comparative Example 3-1; FIG. 17A is a bright-field image, and FIG. 17B is a fluorescent image;
FIG. 18 presents images observed under a phase-contrast microscope, and illustrates a porous structure in Example 4-1; FIG. 18A is a bright-field image, and FIG. 18B is a fluorescent image;
FIG. 19 presents images observed under a phase-contrast microscope, and illustrates a porous structure in Comparative Example 4-1; FIG. 19A is a bright-field image, and FIG. 19B is a fluorescent image;
FIG. 20 is an image of a cultured-cell-comprising substance in Example 5, in which the image was observed under a confocal microscope;

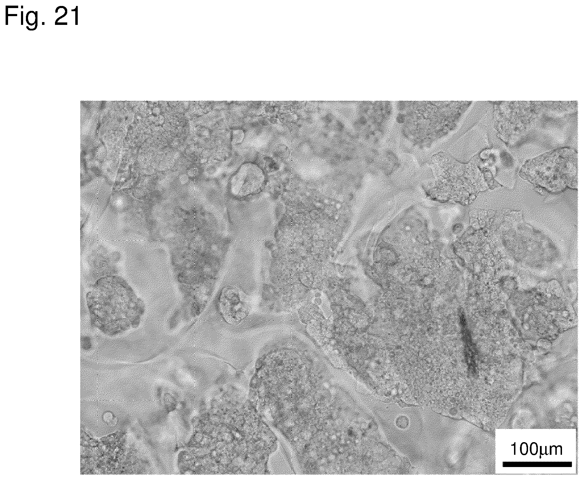

FIG. 21is an image of a cultured-cell-comprising substance in Example 6, in which the image was observed under a confocal microscope;
FIG. 22 is an image of a cultured-cell-comprising substance in Example 7, in which the image was observed under a confocal microscope; and
FIG. 23 is an image of a cultured-cell-comprising substance in Example 8, in which the image was observed under a confocal microscope.
FIG. 24 is an image of a cultured-cell-comprising substance in Example 9, in which the image was observed under a confocal microscope.

### DESCRIPTION OF THE EMBODIMENTS

### 1. Porous Structure

A first embodiment of the present invention is a porous structure. The porous structure in the present embodiment comprises at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the porous structure.

As used herein, the "porous architecture" refers to a three-dimensional architecture having one or more pores. As used herein, the "porous structure" refers to a three-dimensional substance having a porous architecture. A porous structure in the present embodiment is not particularly limited to any use, but is typically used as a cell culture carrier. The porous structure in the present embodiment is advantageous in that, even when formed so as to have high porosity, the porous structure maintains high mechanical strength.

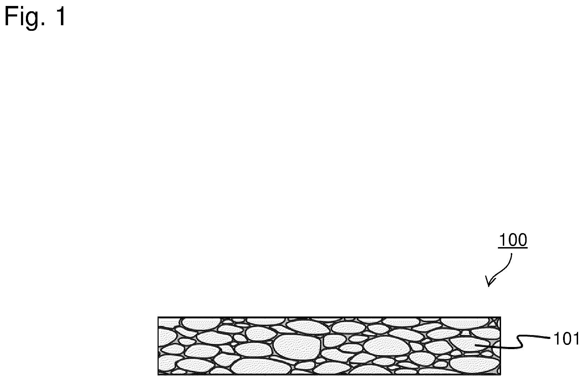

FIG. 1 is a schematic cross-sectional view illustrating an example of a porous structure in the present embodiment. In a porous structure 100 illustrated in the drawing, many pores 101 are formed, and thus, the porous structure 100 has a high specific surface area. A wall portion of a pore 101 has a high specific surface area, and hence, enables many cells to adhere to the wall portion, making an efficient culture possible. In the example illustrated, the pore 101 is generally spherical. The average pore size of the pores is from 5 µm to 1000 µm, particularly preferably from 10 µm to 500 µm. Having the average pore size of the pores within these ranges makes it possible that cells are infiltrated into the pores 101, uniformizing the three-dimensional distribution of the cells, and also retaining the shape of the porous structure stably. The pores 101 which are adjacent to each other may be in communication with each other, thus enabling cells to be spread from one pore to another. The average pore size can be measured by observing the surface portion of the porous structure under a scanning electron microscope.

As used herein, the "porosity" refers to a ratio represented by the following formula: the volume of the void space/(the volume of the solid portions + the volume of the void spaces).

In the example illustrated, the porous structure in the present embodiment is in polyhedral shape, but not limited to a polyhedral shape, and may have any shape selected from the group consisting of a polyhedral shape, generally spherical shape, generally elliptic shape, sheet-like shape, and tubular shape. The thickness of the porous structure is not particularly limited, but is preferably 0.5 mm or more, particularly preferably 1 mm or more. As used herein, the "thickness" of the porous structure means a distance between one face constituting the surface of the porous structure and the face opposed to the surface, that is, the length of a normal line drawn from an arbitrary point on one face to the opposite face. The thickness of the porous structure may be uniform, or may be different from portion to portion in the porous structure, but is preferably uniform.

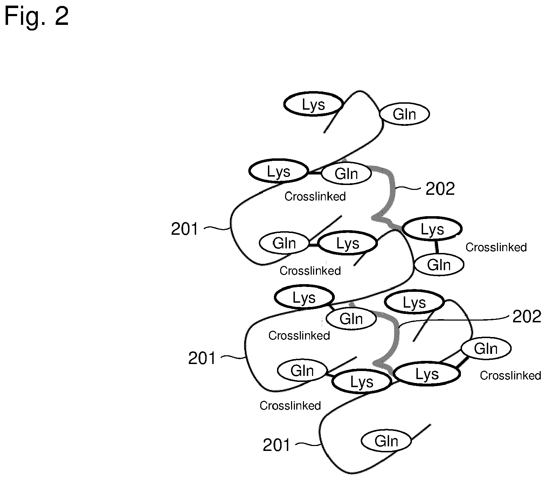

FIG. 2 is a schematic diagram illustrating an example of the molecular architecture of the porous structure in the present embodiment. The porous structure in the present embodiment comprises at least two types of polypeptides, that is, a first polypeptide and a second polypeptide. In the example illustrated, a first polypeptide 201 is crosslinked with another first polypeptide 201 and with a second polypeptide 202. Here, the "first polypeptide" is a polypeptide to become a main backbone of the porous structure, is typically a protein, and is preferably a polypeptide (protein) having at least one of a glutamine residue and/or a lysine residue. The first polypeptide is not particularly limited as long as the polypeptide does not inhibit the activity and proliferation of a cell. The polypeptide can be suitably selected in accordance with the purpose. Particularly desirably, the polypeptide can form a gel, and is such that the gel formed has biodegradability and/or, has adhesiveness to cells or is easily adsorbed by a material adhesive to cells. Examples of such a polypeptide that can be used include a protein selected from the group consisting of collagen, gelatin, fibrin, and elastin. In particular, a biodegradable protein can be suitably used. The molecular weight of the first polypeptide is not particularly limited, but can be from 10,000 to 1,000,000, particularly from 50,000 to 500,000, furthermore from 100,000 to 400,000.

As used herein, the "gel" refers to a polymer having a three-dimensional network architecture or to a swollen product of the polymer, and is typically a hydrogel. As used herein, the "hydrogel" refers to a gel that retains a large amount of water inside the spaces in the network architecture, and that is also referred to as a "hydrous gel". A porous structure is prepared by performing lyophilization or the like to remove a solvent (typically water) from the gel.

The "second polypeptide" is a polypeptide to become a backbone-forming component of the porous structure, is typically a protein, and is preferably a polypeptide (protein) having at least one of a glutamine residue and a lysine residue. More specifically, the second polypeptide preferably has a lysine residue in a case where the first polypeptide has a glutamine residue, and the second polypeptide preferably has a glutamine residue in a case where the first polypeptide has a lysine residue. The second polypeptide is not particularly limited as long as the polypeptide does not inhibit the activity and proliferation of cells. The polypeptide can be suitably selected in accordance with the purpose. Particularly desirably, the polypeptide has biodegradability and/or, has adhesiveness to cells or is a smaller molecule than the first polypeptide. Examples of such a polypeptide that can be used include a protein selected from the group consisting of laminin, fibronectin, thrombin, vimentin, heparin, and vitronectin. In particular, a biodegradable polypeptide (protein) can be suitably used. The molecular weight of the second polypeptide is not particularly limited, but can be from 10 to 1,000,000, particularly from 50,000 to 500,000, furthermore from 100,000 to 300,000.

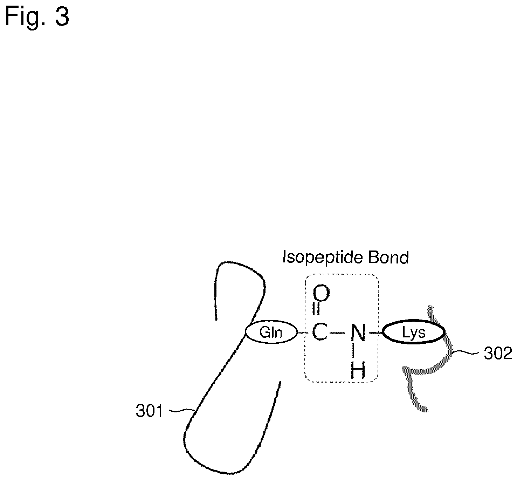

Gln and Lys in FIG. 2 represent a glutamine residue and a lysine residue respectively of an amino acid residue of the first polypeptide or the second polypeptide. In the example illustrated, the first polypeptide and the second polypeptide each have both Gln and Lys, and a glutamine residue and a lysine residue are crosslinked between the first polypeptides and between the first polypeptide and the second polypeptide, thus forming an isopeptide bond. FIG. 3 illustrates a schematic diagram of an example of the isopeptide bond. In the example illustrated in FIG. 3, a glutamine residue on a first polypeptide 301 and a lysine residue on a second polypeptide 302 are crosslinked to form an isopeptide bond. Such an isopeptide bond can be formed, for example, using transglutaminase.

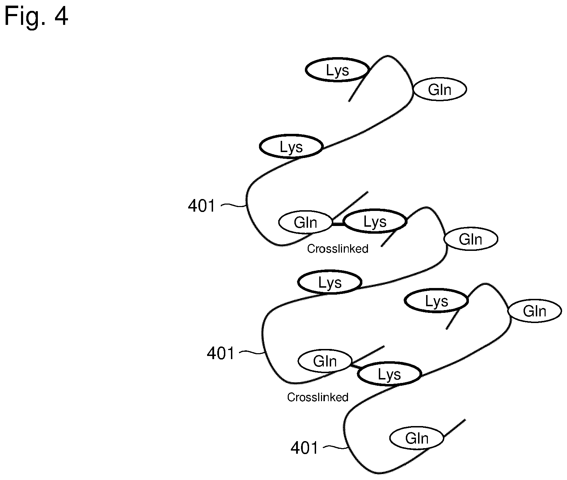

FIG. 4 is related to a conventional porous structure such as described in Non-Patent Literature 1, and is a schematic diagram illustrating an example of the molecular architecture of the porous structure. The example illustrated in FIG. 4 comprises simply one type of polypeptide (401). The polypeptide 401 is desirably a polypeptide to become a main backbone of the porous structure, and is usually and desirably has a given or larger molecular weight. Examples of such a polypeptide include collagen, gelatin, fibrin, and elastin. The molecular weight of the polypeptide 401 is not particularly limited, but can be, for example, from 10,000 to 1,000,000, particularly from 50,000 to 500,000, furthermore from 100,000 to 400,000. That is, the polypeptide 401 comprised in the porous structure as illustrated in FIG. 4 has substantially the same meaning as the "first polypeptide" in a porous structure in the present embodiment. In the example illustrated, the polypeptide 401 has both a glutamine residue (Gln) and a lysine residue (Lys). The action of the transglutaminase causes a crosslinking reaction to form an isopeptide bond between the polypeptides of the same type, thus forming a gel. As used herein, the "gel" refers to a gel that has a three-dimensional network architecture and retains a large amount of water inside the spaces in the network architecture, and that is also referred to as a "hydrous gel". The porous structure is prepared by removing water from the gel by lyophilization or the like.

However, in the example illustrated in FIG. 2, that is, an example of a porous structure in the present embodiment, a gel is formed by a crosslinking reaction caused by a glutamine residue and a lysine residue between different types of polypeptides (the first polypeptide and the second polypeptide) to form an isopeptide bond, in parallel with a crosslinking reaction caused by a glutamine residue and a lysine residue between polypeptides of the same type (the first polypeptides) to form an isopeptide bond. The porous structure in the present embodiment is prepared by performing lyophilization or the like to remove a solvent (typically water) from the gel. Accordingly, a porous structure according to the present invention exists with the first polypeptide and the second polypeptide dispersed overall in the porous structure.

In the case of the porous structure in the present embodiment, it is inferred that, in the crosslinking reaction between the first polypeptides during the formation of a gel, an uncrosslinked glutamine residue or lysine residue is present owing to the steric hindrance due to the conformation, such as the position of the amino acid residue, because the first polypeptide is capable of forming a gel, and a very large molecule forming network. It is conceivable that, in a case where the first polypeptide and the second polypeptide are mixed and crosslinked with transglutaminase, the second polypeptide that is a relatively small molecule enters between the first polypeptides that are very large molecules, and crosslinked with an unreacted amino acid residue, and hence, the points of crosslinking are more than in the case of one type of polypeptide. Accordingly, the porous structure in the present embodiment, formed by two or more types of polypeptides, can be less prone to collapse, and better in shape retention, than a porous structure formed simply by polypeptides of the same type. Thus, the porous structure in the present embodiment can achieve shape retention even if the porous structure has a porosity higher than conventionally. Because the porous structure has a porous architecture, oxygen and nutrients can reach the central part of the structure. For example, in a case where the structure is used as a cell culture carrier, the cells in the central part can be prevented from dying.

As used herein, the "shape retention" refers to the fact that, even when immersed in a culture liquid, buffer liquid, water, or the like, the porous structure is maintained in shape without collapsing to be fragmented or dissolved.

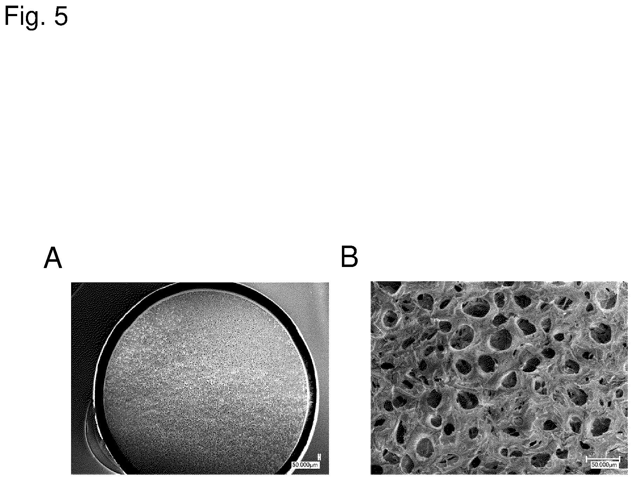

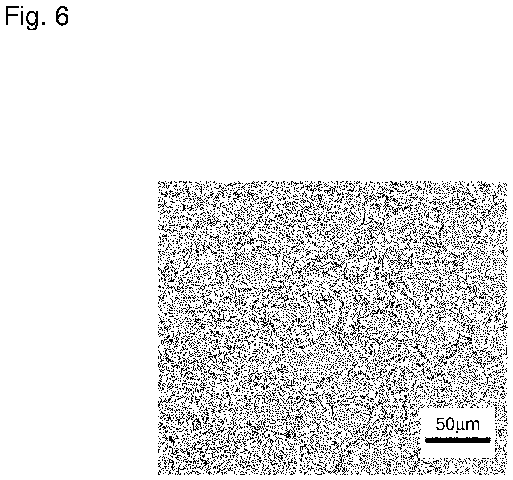

FIG. 5 presents images observed under a microscope, and illustrates an example of the porous structure in the present embodiment. FIG. 5A is an image of the whole porous structure, and FIG. 5B is an enlarged image of FIG. 5A. It can be verified that a porous architecture is substantially uniformly formed overall in the porous structure. FIG. 6 is related to an example of the porous structure in the present embodiment, and illustrates an image of a frozen section observed under a phase-contrast microscope, in which the frozen section was obtained by immersing the porous structure in PBS (-), and then freezing and slicing the porous structure. The fact that the porous architecture was retained even after the structure was immersed in PBS (-) can be verified.

The polypeptide (polypeptide corresponding to the first polypeptide) to be used to form a main backbone of the porous structure in the present embodiment is not limited to one kind but may be a plurality of kinds of polypeptides. For example, two or more kinds of polypeptides selected from the group consisting of collagen, gelatin, fibrin, and elastin may be used. The polypeptide (polypeptide corresponding to the second polypeptide) to be used to become a backbone-forming component of the porous structure in the present embodiment is also not limited to one kind but may be a plurality of kinds of polypeptides. For example, two or more polypeptides selected from the group consisting of laminin, fibronectin, thrombin, vimentin, heparin, and vitronectin may be used.

The porous structure in the present embodiment may further comprise one or more kinds of materials selected from the group consisting of a cell adhesion factor, cell growth factor, and cell differentiation control factor. As used herein, the "cell adhesion factor" means a factor for the adhesion of a cell, and refers to, for example, a substance such as integrin, sarcoglycan, or tenascin. The "cell growth factor" means a factor for the existence, proliferation, growth, or differentiation or the like of a cell, and refers to, for example, a substance such as an epidermal growth factor (EGF), nerve growth factor (NGF), and brain-derived neurotrophic factor (BDNF). The "cell differentiation control factor" refers to a substance such as a neuron differentiation factor (Mash1), astrocyte differentiation factor (Smads), or bone morphogenetic factor (BMP). The cell adhesion factor, cell growth factor, or cell differentiation control factor to be used can be suitably selected in accordance with the purpose, and may be simply of one kind, or may be of two or more kinds.

A technique for adding the cell adhesion factor, cell growth factor, or cell differentiation control factor is not particularly limited, but can be, for example, a technique in which the factor is suspended in a solution of the first polypeptide, and the suspension is filled into the porous structure.

The porous structure in the present embodiment can be used as a cell culture carrier directly in culture. Having a porous architecture means having a large specific surface area, and enables a large number of cells to be cultured. Having a porous architecture also makes it easier for oxygen and nutrients to reach the central part of the cell culture carrier, and thus, can inhibit the death of the cells in the central part. In particular, in an aspect in which a biodegradable material is used, it is also possible that the cells are directly implanted after a cell culture without being separated from the cell culture carrier. A porous structure according to the present invention can be used in the same applications as a usual porous substance, for example, can be used as a packing material for column chromatography.

### 2. Cultured-Cell-Comprising Substance

A second embodiment of the present invention is a cultured-cell-comprising substance. The cultured-cell-comprising substance in the present embodiment is characterized by comprising the porous structure described in the section "1. Porous Structure" and a cell. More specifically, the cultured-cell-comprising substance is obtained by allowing cells to adhere to the porous structure, and culturing the cells in the porous structure. The cultured-cell-comprising substance in the present embodiment is advantageous in that using the porous structure affords a high cell survival rate in the central part.

The kind of the cell is not particularly limited, and any cell may be used in accordance with the purpose. The cell may be, for example, any of a eukaryotic cell, prokaryotic cell, multicellular organism cell, and unicellular organism cell. The cell of one single kind may be used, or the cells of two or more kinds may be used in mixture.

Examples of a eukaryotic cell to be used as the cell include an animal cell, insect cell, plant cell, fungus, and the like. In particular, an animal cell is preferable. As the eukaryotic cell, a free-floating cell or an adhesive cell can be used, and a differentiated cell or an undifferentiated cell may be used. Examples of the differentiated cell include; a liver cell that is the parenchymal cell of the liver; endothelial cells such as a stellate cell, Kupffer cell, vascular endothelial cell, sinusoidal endothelial cell, and corneal endothelial cell; epidermal cells such as a fibroblast, osteoblast, osteoclast, periodontium-derived cell, and epidermal keratinial cell; epithelial cells such as a tracheal epithelial cell, gastrointestinal epithelial cell, uterocervical epithelial cell, and corneal epithelial cell; muscle cells such as a mammary glandular cell, pericyte, smooth muscle cell, and myocardial cell; a renal cell; a pancreas islet cell; a chondrocyte; an osteocyte; a neurocyte; a hybridoma; a hemocyte-derived cell; an ovarian cell; and the like. The undifferentiated cell is not particularly limited, and can be suitably selected in accordance with the purpose. Examples of the undifferentiated cell include: an embryonic stem cell; a pluripotent stem cell such as a mesenchymal stem cell having pluripotency; a unipotent stem cell such as an endothelial precursor cell having unipotency; an iPS cell; and the like. Examples of the prokaryotic cell include eubacteria, archaebacteria, and the like.

A cell culture can be performed, for example, as follows: a porous structure comprising cells fastened inside is immersed in a cell culture medium; and the cells are cultured in an incubator with an environment at 37°C and under 5 vol% CO₂. The amount of the medium to be added can be such an amount that enables at least the whole porous structure to be immersed.

The cultured-cell-comprising substance in the present embodiment is not particularly limited, and can be used for implantation, drug-discovery screening, tissue models, and the like. In particular, in an aspect in which a biodegradable material is used as the porous structure (cell culture carrier), the cells can be directly implanted in an organism without being separated from the cell culture carrier.

### 3. Method for Producing Porous Structure

A third embodiment of the present invention is a method for producing a porous structure. The method in the present embodiment is characterized by comprising: preparing a liquid mixture comprising at least a first polypeptide and a second polypeptide; adding transglutaminase to the liquid mixture and allowing the resulting mixture to react to form a gel; and forming a porous structure from the gel. More specifically, the method in the present embodiment is a method for producing a porous structure described in the section "1. Porous Structure". The terms, detailed conditions, and the like in the present embodiment are the same as in the first embodiment, unless otherwise specified and unless inconsistent.

The method in the present embodiment comprises preparing a liquid mixture comprising at least a first polypeptide and a second polypeptide. A solvent to be comprised in the liquid mixture is not particularly limited as long as the solvent does not affect the properties of a polypeptide very much. PBS (-) is preferably used as the solvent. The concentration of the first-polypeptide solution in the liquid mixture is not particularly limited, but is preferably 0.001 (w/v)% or more and 30 (w/v)% or less, more preferably from 0.5 (w/v)% to 10 (w/v)%. Having the concentration of the first-polypeptide solution within the above-described ranges enables the porous structure to achieve shape retention, and simultaneously maintain a high porosity. The concentration of the second polypeptide is not particularly limited, but is preferably 0.00001 (w/v)% or more and 1 (w/v)% or less, more preferably from 0.0001 (w/v)% to 0.1 (w/v)%. The liquid mixture may be prepared by mixing a solution of the first polypeptide and a solution of the second polypeptide.

The liquid mixture may comprise another component besides the first polypeptide and the second polypeptide. For example, the liquid mixture may comprise at least one of a polypeptide capable of becoming another main backbone and a polypeptide capable of becoming a backbone-forming component. Additionally or alternatively, the liquid mixture may further comprise one or more kinds of materials selected from the group consisting of a cell adhesion factor, cell growth factor, and cell differentiation control factor. The another component is not particularly limited, but can be comprised in the liquid mixture, for example, by suspending the component in the first-polypeptide solution, and then mixing the first-polypeptide solution and the second-polypeptide solution.

The method in the present embodiment comprises subsequently adding transglutaminase to the liquid mixture, and allowing the resulting mixture to react to form a gel. Herein, the "transglutaminase" is not particularly limited as long as the transglutaminase crosslinks a glutamine residue of the polypeptide with a lysine residue to form an isopeptide bond. The transglutaminase can be suitably selected in accordance with the purpose. For example, a transglutaminase such as Factor XIII, TG1, TG2, TG3, TG4, TG5, TG6, or TG7 can be used. The transglutaminase is particularly preferably a transglutaminase derived from a microorganism (for example, actinomyces or the like).

The method in the present embodiment comprises subsequently forming a porous structure from the gel. More specifically, the method comprises removing water from the gel to uncover the network architecture. A specific technique for the present process is not particularly limited as long as the technique forms a porous structure, but the technique can be, for example, lyophilization, foaming by gas, emulsion, or the like.

### 4. Method for Culturing Cells

A fourth embodiment of the present invention is a method for culturing cells. The method in the present embodiment is characterized by comprising: seeding cells in the porous structure described in the section "1. Porous Structure"; and culturing the cells in the porous structure. The terms, detailed conditions, and the like in the present embodiment are the same as in the first embodiment and the second embodiment, unless otherwise specified and unless inconsistent.

The method in the present embodiment comprises seeding cells in the porous structure. A technique for seeding cells on the pore walls of the porous structure is not particularly limited as long as the technique is possible. For example, a possible technique comprises dropping, into the porous structure, a cell suspension having cells suspended in a medium.

The method in the present embodiment comprises subsequently culturing the cells in the porous structure. The cell culture is not particularly limited, but can be performed, for example, as follows: a porous structure comprising cells fastened inside is immersed in a cell culture medium; and the cells are cultured in an incubator with an environment at 37°C and under 5 vol% CO₂. The amount of the medium to be added can be such an amount as enables at least the whole porous structure to be immersed.

### 5. Method for Producing Cultured-Cell-Comprising Substance

A fifth embodiment of the present invention is a method for producing a cultured-cell-comprising substance. The method in the present embodiment is characterized by comprising: seeding cells in the porous structure described in the section "1. Porous Structure"; and culturing the cells in the Porous Structure. The terms, detailed conditions, and the like in the present embodiment are the same as in the first embodiment and the second embodiment, unless otherwise specified and unless inconsistent.

The method in the present embodiment comprises seeding cells in the porous structure. A technique for seeding cells on the pore walls of the porous structure is not particularly limited as long as the technique is possible. For example, a possible technique comprises immersing the porous structure in a cell suspension having cells suspended in a medium.

The method in the present embodiment comprises subsequently culturing the cells in the porous structure. The cell culture is not particularly limited, but can be performed, for example, as follows: a porous structure comprising cells fastened inside is immersed in a cell culture medium; and the cells are cultured in an incubator with an environment at 37°C and under 5 vol% CO₂. The amount of the medium to be added can be such an amount that enables at least the whole porous structure to be immersed.

### 6. Hydrogel structure

A sixth embodiment of the present invention is a hydrogel structure. The hydrogel structure in the present embodiment is characterized in comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the structure.

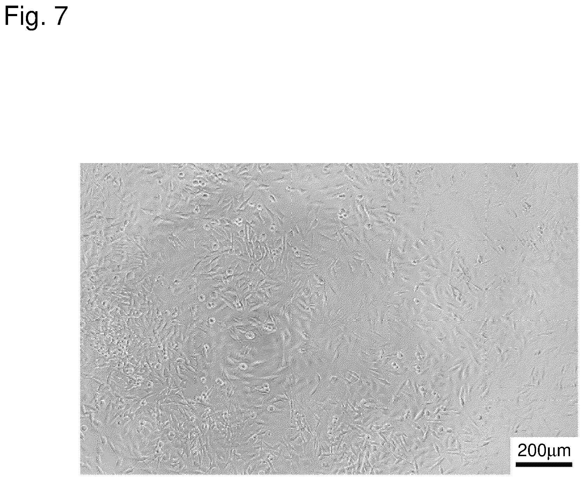

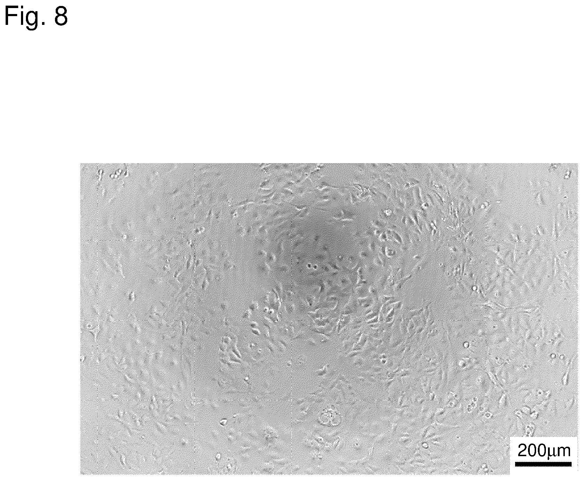

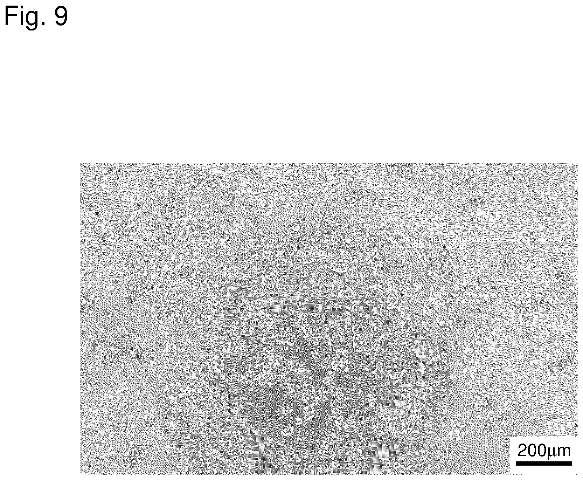

The porous structure of the first embodiment of the present invention may be prepared by removing a solvent (water) from a hydrogel in which the first polypeptide and the second polypeptide are dispersed throughout the structure by, for example, freeze-drying or the like. However, the hydrogel without being removed the solvent may also be used for cell culture in the form of the gel. FIGs. 7 to 9 show microscopic images of cells seeded and cultured on hydrogel structures without forming porous structures. The hydrogel structures of FIGs. 7 to 9 are structures formed by using gelatin as a first protein and laminin as a second protein, and adding transglutaminase to a mixture containing these proteins to react. FIG. 7 presents an image obtained by culturing NIH/3T3 cells (Clone 5611, JCRB Cell Bank, hereinafter also referred to as "3T3"), FIG. 8 presents an image obtained by culturing HepG2 cells (human hepatoma-derived cell line), and FIG. 9 presents an image obtained by culturing PC12 cells (rat adrenal pheochromocytoma). It is shown that many cells can adhere to the gel and be cultured because the hydrogel structures are made of polypeptides that cells easily adhere to. It is also shown that the cells can adhere to and cultured on the gel even if the cell type is changed.

The hydrogel structure of the present embodiment may be used for cell culture as it is, but may also be used as a precursor of the porous structure. The step of forming the porous structure from the precursor comprising the hydrogel structure or consisting of the hydrogel structure can be performed by removing the solvent (water) from the gel by, for example, freeze-drying or the like.

### 7. Method for producing hydrogel structure

A seventh embodiment of the present invention is a method for producing a hydrogel structure. The method of the present embodiment is characterized in comprising: preparing a liquid mixture comprising at least a first polypeptide and a second polypeptide; and adding transglutaminase to the liquid mixture and allowing the resulting mixture to react to form a gel. The method according to the present embodiment is particularly suitable for producing a hydrogel structure according to the section "6. Hydrogel structure". More specifically, the method of the present embodiment is a method in which a step of forming a porous structure from a hydrogel is omitted from the method described in "3. Method for Producing a Porous Structure". As described above, the hydrogel structure produced by such a method can be used for cell culture even in a form of a hydrogel structure rather than a porous structure, particularly when a polypeptide having high cell adhesion is used. Alternatively, the hydrogel structure may be used as a precursor of a porous structure.

### EXAMPLES

Below, the present invention will be specifically described with reference to Examples, but the description below is not intended to limit the scope of the present invention to the scope of the Examples.

### [Example 1-1]

A porous structure was produced using an acid-treated gelatin as the first polypeptide and using laminin as the second polypeptide.

### (1) Preparation of gel precursor

Gelatin (gelatin LET-NP250, manufactured by Nitta Gelatin Inc., having a molecular weight of 100,000 to 300,000) was dissolved in PBS (-) (Dulbecco's Phosphate Buffered Saline: DPBS (1×), manufactured by Gibco) mixed with fluorescent beads (Fluoro-Max colored green aqueous fluorescent particles, 5 µm, manufactured by Thermo Fisher Scientific Inc.) at a concentration of 0.05%. A solution of gelatin at a concentration of 0.5 (w/v)% was thus prepared.

### (2) Formation of hydrogel

To 100 µL of the gelatin solution, 1 µL of 0.5 mg/mL laminin (iMatrix-511, manufactured by Nippi, Inc.) solution was added, and the resulting solution was mixed with stirring using a vortex mixer (LSE, manufactured by Corning Inc.). Then, 1 µL of a solution of transglutaminase (Bacterial transglutaminase, manufactured by Zedira GmbH) adjusted to 25 U/mL in PBS (-) was added, and the resulting solution was mixed with stirring using the vortex mixer. Into a 35 mm dish, 30 µL of the gelatin solution comprising laminin and transglutaminase in mixture was dropped in dome-like form. The gelatin solution dropped into the dish was put in the incubator under the condition at 37°C and under 5 vol% CO₂ for 4 hours, and then was left to stand at 4°C for one day to form a hydrogel.

### (3) Production of porous structure

The hydrogel was frozen using a deep freezer (CLN-2300CWE, manufactured by Nippon Freezer Co., Ltd.) at -150°C for 30 minutes, and then dried using a small plasma device (PM100, manufactured by Yamato Scientific Co., Ltd.) under reduced pressure at 5 Pa or less for two hours to obtain a porous structure.

### (4) Evaluation of shape retention

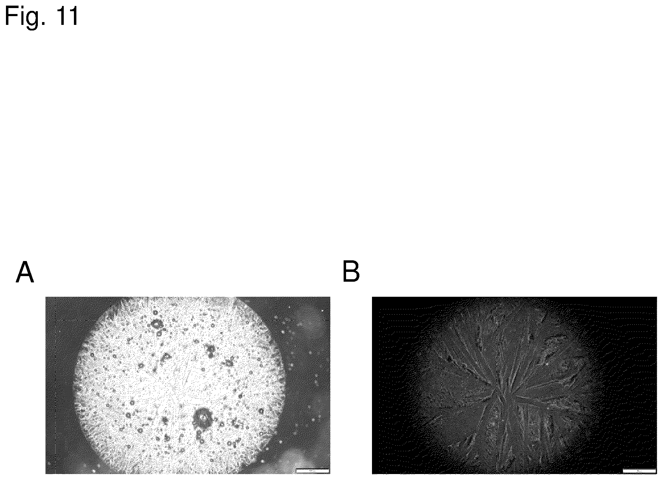

To the dish comprising the porous structure obtained in (3), 3 mL of PBS (-) was added, so that the porous structure was immersed in the PBS (-). Whether the porous structure achieved shape retention was evaluated. FIG. 11 presents images of a porous structure in Example 1-1, in which the images were observed under a phase-contrast microscope (CKX53, manufactured by Olympus Corporation). FIG. 11A is a bright-field image, and FIG. 11B is a fluorescent image. As illustrated in FIG. 11A, it has been verified that, even after being immersed in PBS (-), the porous structure did not collapse, and achieved shape retention. As illustrated in FIG. 11B, it was observed that the fluorescent beads mixed in the porous structure did not flow out, allowing the structure to achieve shape retention.

### [Comparative Example 1-1]

A porous structure was produced using an acid-treated gelatin as the first polypeptide without using the second polypeptide in Example 1-1.

### (1) Preparation of gel precursor

Gelatin was dissolved in PBS (-) mixed with fluorescent beads at a concentration of 0.05%, and a solution of gelatin at a concentration of 0.5 (w/v)% was thus prepared.

### (2) Formation of hydrogel

To 100 µL of the gelatin solution, 1 µL of a solution of transglutaminase adjusted to 25 U/mL in PBS (-) was added, and the resulting solution was mixed with stirring using the vortex mixer. Into a 35 mm dish, 30 µL of the gelatin solution comprising transglutaminase in mixture was dropped in dome-like form. The gelatin solution dropped into the dish was put in the incubator under the condition at 37°C and under 5 vol% CO₂ for 4 hours, and then was left to stand at 4°C for one day to form a hydrogel.

### (3) Production of porous structure

The hydrogel was frozen using a deep freezer at -150°C for 30 minutes, and then dried using a small plasma device under reduced pressure at 5 Pa or less for two hours to obtain a porous structure.

### (4) Evaluation of shape retention

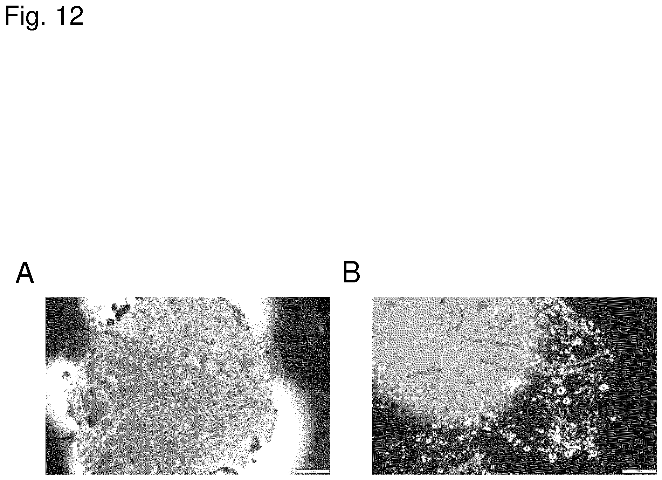

To the dish comprising the porous structure obtained in (3), 3 mL of PBS (-) was added, so that the porous structure was immersed in the PBS (-) to evaluate whether the porous structure successfully achieved shape retention. The form of the porous structure that had been immersed in PBS (-) was observed to evaluate the shape retention. FIG. 12 presents images of a porous structure in Comparative Example 1-1, in which the images were observed under a phase-contrast microscope. FIG. 12A illustrates a bright-field image, and FIG. 12B illustrates a fluorescent image. As illustrated in FIG. 12A, the porous structure collapsed immediately after being immersed in PBS (-), and thus, did not achieve shape retention. As illustrated in FIG. 12B, it was observed that the fluorescent beads mixed in the porous structure flowed out, and that the cell culture carrier collapsed.

The results in Example 1-1 have verified that, even after the porous structure was immersed in a buffer liquid, culture liquid, or the like, the shape retention was achieved by crosslinking the first polypeptide with the second polypeptide to produce a porous structure having a porous architecture. The results in Comparative Example 1-1 have revealed that the porous structure not comprising the second polypeptide collapsed when immersed in a buffer liquid or the like, and that shape retention was difficult.

### [Example 2-1]

A porous structure having a porous architecture was produced using an alkali-treated gelatin as the first polypeptide and using laminin as the second polypeptide.

### (1) Preparation of gel precursor

Gelatin (BeMatrix gelatin LS-H, manufactured by Nitta Gelatin Inc., having a molecular weight of 100,000 to 300,000) was dissolved in 100 µL of a 0.5 mg/mL laminin solution mixed with fluorescent beads at a concentration of 0.05%. A solution of gelatin at a concentration of 1.0 (w/v)% was thus prepared.

### (2) Formation of hydrogel

To 100 µL of the gelatin solution, 1 µL of a solution of transglutaminase adjusted to 25 U/mL in PBS (-) was added, and the resulting solution was mixed with stirring using the vortex mixer. Then, into a 35 mm dish, 30 µL of the gelatin solution comprising laminin and transglutaminase in mixture was dropped in dome-like form. The gelatin solution dropped into the dish was put in the incubator under the condition at 37°C and under 5 vol% CO₂ for 4 hours, and then was left to stand at 4°C for one day to form a hydrogel.

### (3) Production of porous structure

The hydrogel was frozen using a deep freezer at -150°C for 30 minutes, and then dried using a small plasma device under reduced pressure at 5 Pa or less for two hours to obtain a porous structure having a porous architecture.

### (4) Evaluation of shape retention

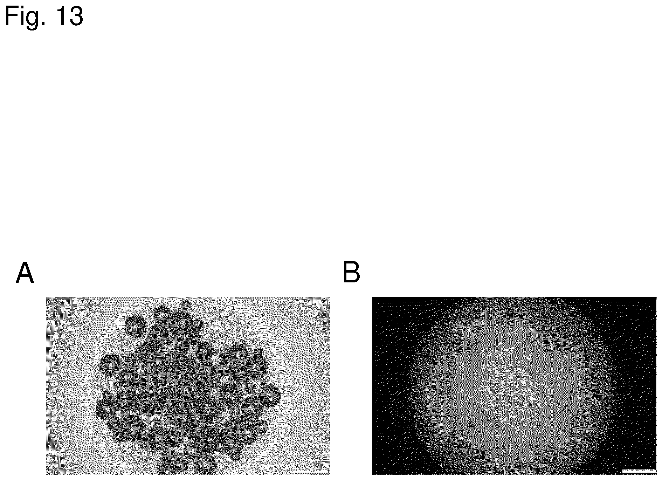

To the dish comprising the porous structure obtained in (3), 3 mL of PBS (-) was added, so that the porous structure was immersed in the PBS (-). Whether the porous structure achieved shape retention was evaluated. The porous structure was immersed in PBS (-), and left to stand for one day in an incubator (a CO₂ incubator KM-CC17RU2, manufactured by Panasonic Corporation) with an environment at 37°C and under 5 vol% CO₂. The form of the structure was observed to evaluate the shape retention. FIG. 13 presents images of a porous structure in Example 2-1, in which the images were observed under a phase-contrast microscope. FIG. 13A is a bright-field image, and FIG. 13B is a fluorescent image. The round substance taking up a large part of the whole image in FIG. 13A is a porous structure having a porous architecture, in which the structure was immersed in PBS (-). Air bubbles that leaked in during the immersion in PBS (-) can be seen overall. These bubbles were retained even after being left to stand for one day, making it possible to verify that the porous architecture did not collapse, and achieved shape retention. As illustrated in FIG. 13B, fluorescent beads were observed in the whole porous structure. It was observed that the fluorescent beads did not flow out, allowing the structure to achieve shape retention.

### [Example 2-2]

A porous structure was produced using an alkali-treated gelatin as the first polypeptide and using fibronectin as the second polypeptide.

### (1) Preparation of gel precursor

Gelatin was dissolved in 100 µL of a solution of 0.5 mg/mL fibronectin (Fibronectin human plasma, manufactured by Sigma-Aldrich, having a molecular weight of 210,000 to 250,000) mixed with fluorescent beads at a concentration of 0.05%. A solution of gelatin at a concentration of 1.0 (w/v)% was thus prepared.

### (2) Formation of hydrogel

To 100 µL of the gelatin solution, 1 µL of a solution of transglutaminase adjusted to 25 U/mL in PBS (-) was added, and the resulting solution was mixed with stirring using the vortex mixer. Then, into a 35 mm dish, 30 µL of the gelatin solution comprising fibronectin and transglutaminase in mixture was dropped in dome-like form. The gelatin solution dropped into the dish was put in the incubator under the condition at 37°C and under 5 vol% CO₂, and then was left to stand at 4°C for one day to form a hydrogel.

### (3) Production of porous structure

The hydrogel was frozen using a deep freezer at -150°C for 30 minutes, and then dried using a small plasma device under reduced pressure at 5 Pa or less for two hours to obtain a porous structure.

### (4) Evaluation of shape retention

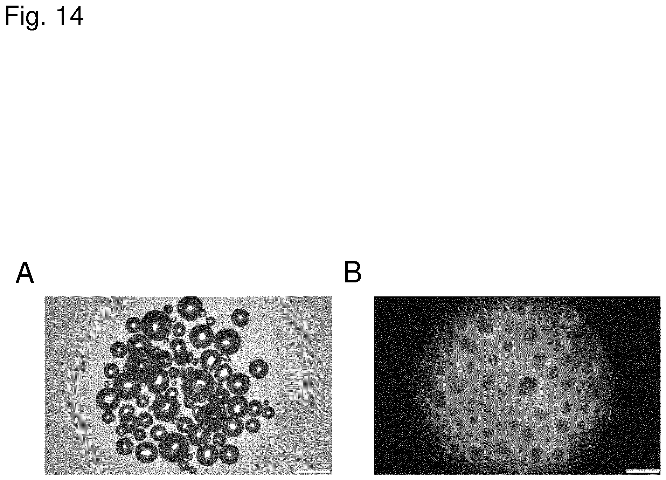

To the dish comprising the porous structure obtained in (3), 3 mL of PBS (-) was added, so that the porous structure was immersed in the PBS (-). Whether the porous structure achieved shape retention was evaluated. The form of the porous structure that had been immersed in PBS (-) and left to stand in an incubator for one day was observed to evaluate the shape retention. FIG. 14 presents images of a porous structure in Example 2-2, in which the images were observed under a phase-contrast microscope. FIG. 14A is a bright-field image, and FIG. 14B is a fluorescent image. The round substance takes up a large part of the whole image in FIG. 14A is a porous structure having a porous architecture, in which the structure was immersed in PBS (-). Air bubbles that leaked in during the immersion in PBS (-) can be seen overall. These bubbles were retained even after being left to stand for one day, making it possible to verify that the porous architecture did not collapse, and achieved shape retention. As illustrated in FIG. 14B, fluorescent beads were observed in the whole porous structure. It was observed that the fluorescent beads did not flow out, allowing the structure to achieve shape retention.

### [Comparative Example 2-1]

A porous structure was produced using an alkali-treated gelatin as the first polypeptide without using the second polypeptide in Example 2-1 or 2-2.

### (1) Preparation of gel precursor

Gelatin was dissolved in PBS (-) mixed with fluorescent beads at a concentration of 0.05%, and a solution of gelatin at a concentration of 1.0 (w/v)% was thus prepared.

### (2) Formation of hydrogel

To 100 µL of the gelatin solution, 1 µL of a solution of transglutaminase adjusted to 25 U/mL in PBS (-) was added, and the resulting solution was mixed with stirring using the vortex mixer. Into a 35 mm dish, 30 µL of the gelatin solution comprising transglutaminase in mixture was dropped in dome-like form. The gelatin solution dropped into the dish was put in the incubator under the condition at 37°C and under 5 vol% CO₂, and then was left to stand at 4°C for one day to form a hydrogel.

### (3) Production of porous structure

The hydrogel was frozen using a deep freezer at -150°C for 30 minutes, and then dried using a lyophilizer under reduced pressure at 5 Pa or less for two hours to obtain a porous structure having a porous architecture.

### (4) Evaluation of shape retention

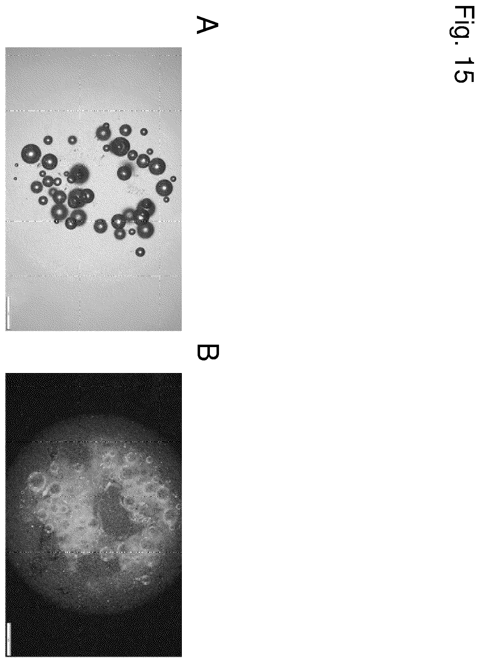

To the dish comprising the porous structure obtained in (3), 3 mL of PBS (-) was added, so that the porous structure was immersed in the PBS (-). Whether the porous structure achieved shape retention was evaluated. The form of the porous structure that had been immersed in PBS (-) and left to stand in an incubator for one day was observed to evaluate the shape retention. FIG. 15 presents images of a porous structure in Comparative Example 2-1, in which the images were observed under a phase-contrast microscope. FIG. 15A is a bright-field image, and FIG. 15B is a fluorescent image. In the image in FIG. 15A, bubbles remained just partially in the porous structure. It was observed that the porous structure dissolved, and did not achieve shape retention. As illustrated in FIG. 15B, it was observed that the fluorescent beads remained partially in the porous structure, but that many of the fluorescent beads came off, and that the field of view is dark. It was observed that, in the porous structure, the portions that dissolved are represented simply by the pale light of the background of the fluorescence. These results have verified that the porous structure in Comparative Example 2-1 did not achieve shape retention.

### [Example 3-1]

A porous structure having a porous architecture was produced using fibrin as the first polypeptide and using laminin as the second polypeptide.

### (1) Preparation of gel precursor

Fibrinogen (Fibrinogen from bovine plasma, manufactured by Sigma-Aldrich) was dissolved in PBS (-) mixed with fluorescent beads at a concentration of 0.05%. A solution of fibrinogen at a concentration of 0.5 (w/v)% was thus prepared. To 100 µL of the fibrinogen solution, 1 µL of a solution of thrombin (Thrombin from bovine plasma, manufactured by Sigma-Aldrich) adjusted to 2 U/mL in PBS (-) was added, and the resulting solution was mixed with stirring using a vortex mixer. A solution of fibrin at a concentration of 0.5 (w/v)% was thus prepared.

### (2) Formation of hydrogel

To 100 µL of the fibrin solution, 1 µL of a 0.5 mg/mL laminin solution was added, and the resulting solution was mixed with stirring using the vortex mixer. Then, 1 µL of a solution of transglutaminase adjusted to 25 U/mL in PBS (-) was added, and the resulting solution was mixed with stirring using the vortex mixer. Into a 35 mm dish, 30 µL of the fibrin solution comprising laminin and transglutaminase in mixture was dropped in a dome-like form. The gelatin solution dropped into the dish was put in the incubator under the condition at 37°C and under 5 vol% CO₂ for 4 hours, and then was left to stand at 4°C for one day to form a hydrogel.

### (3) Production of porous structure

The hydrogel was frozen using a deep freezer at -150°C for 30 minutes, and then dried using a lyophilizer under reduced pressure at 5 Pa or less for two hours to obtain a porous structure having a porous architecture.

### (4) Evaluation of shape retention

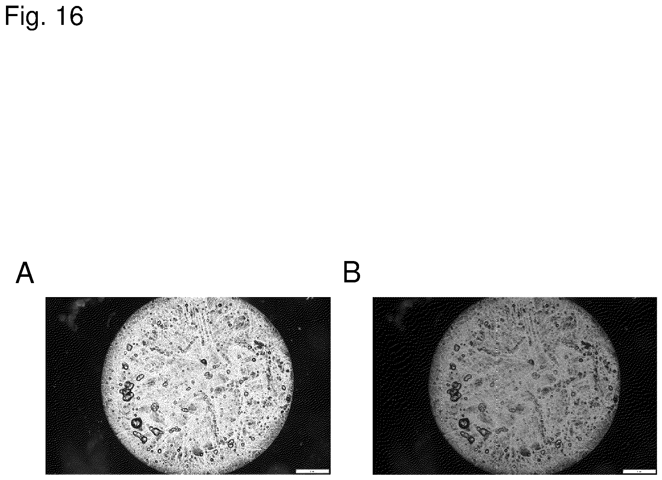

To the dish comprising the porous structure obtained in (3), 3 mL of PBS (-) was added, so that the porous structure was immersed in the PBS (-). Whether the porous structure achieved shape retention was evaluated. The form of the porous structure that had been immersed in PBS (-) was observed to evaluate the shape retention. FIG. 16 presents images of a porous structure in Example 3-1, in which the images were observed under a phase-contrast microscope. FIG. 16A is a bright-field image, and FIG. 16B is a fluorescent image. As illustrated in FIG. 16A, it has been verified that, even after being immersed in PBS (-), the porous structure did not collapse, and achieved shape retention. As illustrated in FIG. 16B, fluorescent beads were observed in the whole porous structure. It was observed that the fluorescent beads did not flow out, allowing the porous structure to achieve shape retention.

### [Comparative Example 3-1]

A porous structure having a porous architecture was produced using fibrin as the first polypeptide without using the second polypeptide in Example 3-1.

### (1) Preparation of gel precursor

Fibrinogen was dissolved in PBS (-) mixed with fluorescent beads at a concentration of 0.05%, and a solution of fibrinogen at a concentration of 0.5 (w/v)% was thus prepared. To 100 µL of the fibrinogen solution, 1 µL of a solution of thrombin adjusted to 2 U/mL in PBS (-) was added, and the resulting solution was mixed with stirring using the vortex mixer. A solution of fibrin at a concentration of 0.5 (w/v)% was thus prepared.

### (2) Formation of hydrogel

To 100 µL of the fibrin solution, 1 µL of a solution of transglutaminase adjusted to 25 U/mL in PBS (-) was added, and the resulting solution was mixed with stirring using the vortex mixer. Then, into a 35 mm dish, 30 µL of the fibrin solution comprising transglutaminase in mixture was dropped in dome-like form. The fibrin solution dropped into the dish was put in the incubator under the condition at 37°C and under 5 vol% CO₂ for 4 hours, and then was left to stand at 4°C for one day to form a hydrogel.

### (3) Production of porous structure

The hydrogel was frozen using a deep freezer at -150°C for 30 minutes, and then dried using a lyophilizer under reduced pressure at 5 Pa or less for two hours to obtain a porous structure.

### (4) Evaluation of shape retention

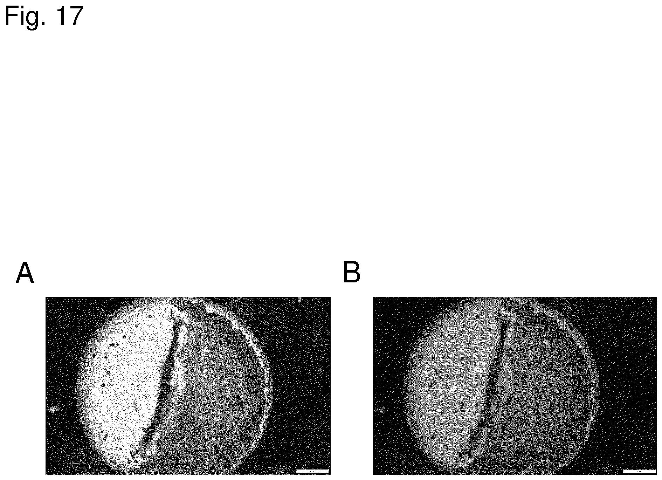

To the dish comprising the porous structure obtained in (3), 3 mL of PBS (-) was added, so that the porous structure was immersed in the PBS (-). Whether the porous structure achieved shape retention was evaluated. FIG. 17 presents images of the form of the porous structure that had been immersed in PBS (-), in which the images were observed under a phase-contrast microscope. FIG. 17A is a bright-field image, and FIG. 17B is a fluorescent image. As illustrated in FIG. 17A, the porous structure was peeled off from the dish and collapsed immediately after being immersed in PBS (-), thus failing to achieve shape retention. As illustrated in FIG. 17B, the portion where the porous structure was present, and where thick fluorescence was observable is approximately half of the image. It was observed that, in the porous structure, the portions that peeled off and collapsed are represented simply by the pale light of the background of the fluorescence. These results have verified that the porous structure in Comparative Example 3-1 did not achieve shape retention.

### [Example 4-1]

A porous structure having a porous structure was prepared using collagen as the first polypeptide and using laminin as the second polypeptide.

### (1) Preparation of gel precursors

One PBS tablet (PBS (Phosphate Buffered Salts) Tablets, manufactured by Takara Bio Co., LTD.) was dissolved in pure water 10mL and filtrated through a filter having an average pore diameter of 0.2 µm (Minisart Syringe Filter 175497K, manufactured by Sartorius AG) to prepare PBS solutions 10 times more concentrated than usual. A collagenous Type I solution (Collagen Type I, 10.21mg/mL, manufactured by Corning), a reconstitution buffer solution (for Cellmatrix, manufactured by Nitta Gelatin Inc.), and the 10-fold concentration PBS comprising 0.05% fluorescent beads were miced at a ratio of 8:1:1. After mixing, the mixture was diluted 2-fold with PBS(-) (Dulbecco's Phosphate Buffered Saline DPBS (1×), manufactured by Gibco) to prepare a collagenous solution.

### (2) Formation of hydrogel

To 100µL of the collagen solution, 1 µL of a 0.5mg/mL laminin solution was added, and the resulting solution was mixed by pipetting. Then, 1 µL of a solution of transglutaminase adjusted to 25 U/mL in PBS (-) was added, and the resulting solution was mixed by pipetting. Into a 35 mm dish, 30 µL of the collagen solution comprising laminin and transglutaminase in mixture was dropped in a dome-like form. The collagen solution dropped into the dish was put in the incubator under the condition at 37°C and under 5 vol% CO₂ for 4 hours, and then was left to stand at 4°C for one day to form a hydrogel.

### (3) Preparation of porous structures

The hydrogel was frozen using a deep freezer at -150°C for 30 minutes, and then dried using a lyophilizer under reduced pressure at 5 Pa or less for two hours to obtain a porous structure having a porous architecture.

### (4) Evaluation of shape retention

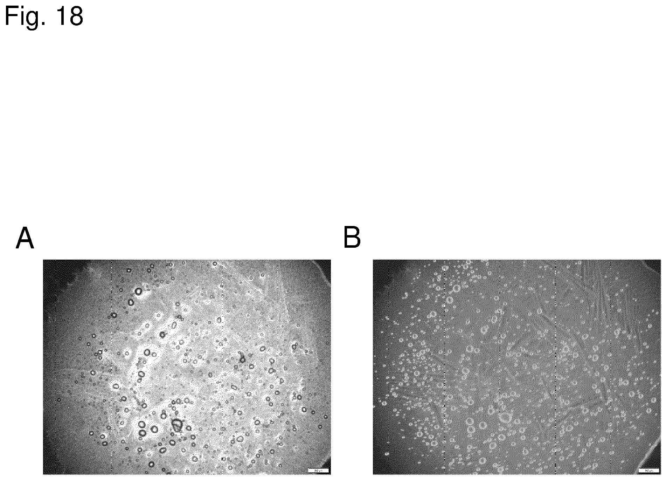

To the dish comprising the porous structure obtained in (3), 3 mL of PBS (-) was added, so that the porous structure was immersed in the PBS (-). Whether the porous structure achieved shape retention was evaluated. The form of the porous structure that had been immersed in PBS (-) was observed to evaluate the shape retention. FIG. 18 presents images of a porous structure in Example 4-1, in which the images were observed under a phase-contrast microscope. FIG. 18A is a bright-field image, and FIG. 18B is a fluorescent image. As illustrated in FIG. 18A, it has been verified that, even after being immersed in PBS (-), the porous structure did not collapse, and achieved shape retention. As illustrated in FIG. 18B, fluorescent beads were observed in the whole porous structure. It was observed that the fluorescent beads did not flow out, allowing the porous structure to achieve shape retention.

### [Comparative Example 4-1]

A porous structure having a porous architecture was produced using collagen as the first polypeptide without using the second polypeptide in Example 4-1.

### (1) Preparation of gel precursors

One PBS tablet was dissolved in pure water 10mL and filtrated through a filter having an average pore diameter of 0.2 µm to prepare PBS solutions 10 times more concentrated than usual. A collagenous Type I solution, a reconstitution buffer solution and the A 10-fold concentration PBS comprising 0.05% fluorescent beads were mixed at a ratio of 8:1:1. After mixing, the mixture was diluted 2-fold with PBS(-) to prepare a collagenous solution.

### (2) Formation of hydrogel

To 100µL of the collagen solution, 1 µL of a solution of transglutaminase adjusted to 25 U/mL in PBS (-) was added, and the resulting solution was mixed by pipetting. Into a 35 mm dish, 30 µL of the collagen solution comprising transglutaminase in mixture was dropped in a dome-like form. The collagen solution dropped into the dish was put in the incubator under the condition at 37°C and under 5 vol% CO₂ for 4 hours, and then was left to stand at 4°C for one day to form a hydrogel.

### (3) Preparation of porous structures

The hydrogel was frozen using a deep freezer at -150°C for 30 minutes, and then dried using a lyophilizer under reduced pressure at 5 Pa or less for two hours to obtain a porous structure having a porous architecture.

### (4) Evaluation of shape retention

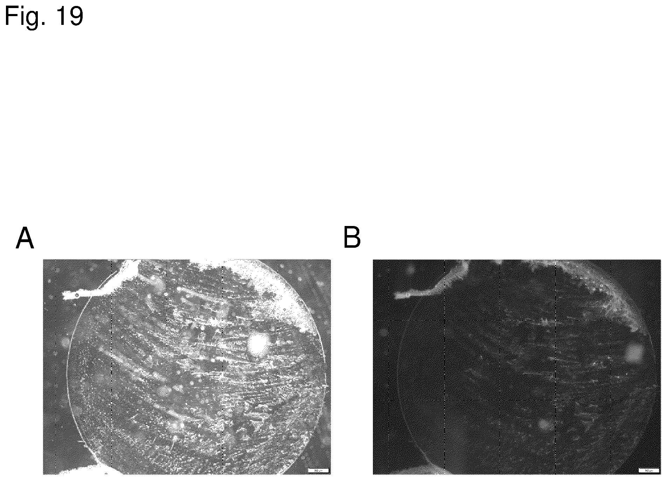

To the dish comprising the porous structure obtained in (3), 3 mL of PBS (-) was added, so that the porous structure was immersed in the PBS (-). Whether the porous structure achieved shape retention was evaluated. FIG. 19 presents images of the form of the porous structure that had been immersed in PBS (-), in which the images were observed under a phase-contrast microscope. FIG. 19A is a bright-field image, and FIG. 19B is a fluorescent image. As illustrated in FIG. 19A, the porous structure was peeled off from the dish and collapsed immediately after being immersed in PBS (-), thus failing to achieve shape retention. In a image in FIG. 19A, it was observed that the remaining porous structure was only a part of the upper portion of the image, and the porous structure dissolved and could not be maintained in shape. As illustrated in FIG. 19B, the portion where the porous structure was present, and where thick fluorescence was observable is approximately half of the image. It was observed that, in the porous structure, the portions that peeled off and collapsed are represented simply by the pale light of the background of the fluorescence. Similarly, in the fluorescence image in FIG. 19B, it was observed that the porous structural member that can be observed with high fluorescence was present only in a part of the upper portion of the image. These results have verified that the porous structure in Comparative Example 3-1 did not achieve shape retention.

In the above-described Examples and Comparative Examples, fluorescent beads were mixed in to increase the visibility of the porous structure. A series of evaluations were performed after verifying that neither mixing fluorescent beads nor mixing no fluorescent beads affected the production of a porous structure, nor affected the results of evaluation of shape retention. The results in Examples 1-1, 2-1, 2-2, 3-1 and 4-1 have verified that, even after the porous structure was immersed in a buffer liquid, culture liquid, or the like, the shape retention of the porous structure was achieved by crosslinking the first polypeptide with the second polypeptide to produce a porous structure. The results in Comparative Examples 1-1, 2-1, 3-1 and 4-1 have revealed that the porous structure not comprising the second polypeptide collapsed when immersed in a buffer liquid or the like, and that shape retention was difficult.

### [Example 5]

A porous structure was produced using an acid-treated gelatin as the first polypeptide and using laminin as the second polypeptide. The porous structure produced was used as a cell culture carrier, in which NIH/3T3 cells (Clone 5611, JCRB Cell Bank, hereinafter referred to as "3T3") were seeded to produce a cultured-cell-comprising substance.

### (1) Preparation of gel precursor

The gelatin was dissolved, and a solution of gelatin at a concentration of 1.0 (w/v)% was thus prepared.

### (2) Formation of hydrogel

To 100 µL of the gelatin solution, 1 µL of a 0.5 mg/mL laminin solution was added, and the resulting solution was mixed with stirring using the vortex mixer. Then, 1 µL of a solution of transglutaminase adjusted to 25 U/mL in PBS (-) was added, and the resulting solution was mixed with stirring using the vortex mixer. Into a 35 mm dish, 30 µL of the gelatin solution comprising laminin and transglutaminase in mixture was dropped in dome-like form. The gelatin solution dropped into the dish was left to stand at 4°C for one day to form a hydrogel.

### (3) Production of porous structure

The hydrogel was frozen using a deep freezer at -150°C for 30 minutes, and then dried using a small plasma device under reduced pressure at 5 Pa or less for two hours to obtain a porous structure.

### (4) Culture of NIH/3T3 Cells

In an incubator, NIH/3T3 cells were cultured in a 100 mm dish for 72 hours, using a Dulbecco's Modified Eagle Medium (DMEM (1×), manufactured by Thermo Fisher Scientific Inc., hereinafter referred to as "DMEM") comprising 10 mass% fetal bovine serum (hereinafter referred to as "FBS") and 1 mass% antibiotic (Antibiotic-Antimycotic Mixed Stock Solution (100×), manufactured by Nacalai Tesque, Inc.).

### (5) Production of NIH/3T3 Cell Suspension

To the dish, 5 mL of PBS (-) was added, and the PBS (-) was removed by suction using an aspirator, so that the surface was washed. After the washing operation with PBS (-), 2 mL of a 0.05% trypsin-0.05% EDTA solution (manufactured by Life Technologies Corp) was added to the dish, the resulting solution was warmed in an incubator for 5 minutes, and the cells were peeled off from the dish. The fact that the cells were peeled off was verified under a phase-contrast microscope, and then, 2 mL of FBS-comprising DMEM was added to the dish to deactivate the trypsin. From the dish, the cell suspension was transferred to one 15 mL centrifuge tube, and centrifuged with a centrifugal (H-19FM, manufactured by KOKUSAN Co., Ltd., 200 × g, 3 minutes). The supernatant was removed using an aspirator. After the removal, 2 mL of FB S-comprising DMEM was added to the centrifuge tube, and gently pipetted. The cells were dispersed to obtain a cell suspension. Into an Eppendorf tube, 100 µL of the cell suspension was taken out, the sample was sucked into a cassette (Vial-Cassette, manufactured by Chemometec A/S), and the number of cells was measured using a cell counter (NucleoCounter NC-3000, manufactured by Chemometec A/S) to determine the number of cells in the liquid. Part of the cell suspension was transferred to a 15 mL centrifuge tube, and centrifuged (200 × g, 3 minutes). The supernatant was removed using a pipette. DMEM was added to obtain a cell suspension having a cell concentration of 5 × 10⁶ cells/mL.

### (6) Production of cultured-cell-comprising substance

To the porous structure produced in (3), 30 µL of the cell suspension produced in (5) was dropped to produce a cultured-cell-comprising substance. To the cells, 2 mL of DMEM comprising 10 mass% FBS and 1 mass% antibiotic was added, and the resulting mixture was placed in an incubator with an environment at 37°C and under 5 vol% CO₂. The cells were cultured for two days.

### (7) Evaluation of cell culture

To the dish comprising the cultured-cell-comprising substance obtained in (6), 0.5 µL of an orange fluorescence dye (Cell Tracker Orange, manufactured by Thermo Fisher Scientific Inc.) was added, and the cells were stained. After the staining, the cells were observed using a confocal microscope (FV10, manufactured by Olympus Corporation), and whether the cells were spread inside the porous structure was evaluated.

### (8) Results of evaluation of cell culture

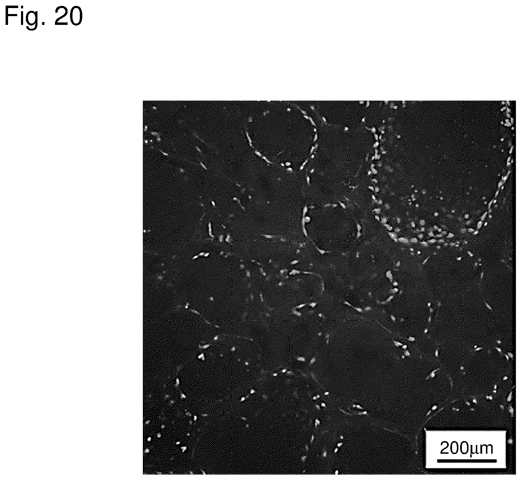

The cultured-cell-comprising substance stained was observed to evaluate the cell culture. FIG. 20 presents an image observed under a confocal microscope. As illustrated in the drawing, it has been verified that the cells spread along the internal architecture of the porous structure.

### [Example 6]

A cultured-cell-comprising substance in the present Example was produced by the technique in accordance with Example 5 except that HepG2 cells (human-liver-cancer-derived cell lines) were used in place of the NIH/3T3 cells.

### (1) Culture of HepG2 cells

In an incubator, the HepG2 cells were cultured in a 100 mm dish for 72 hours, using DMEM comprising 10 mass% FBS and 1 mass% antibiotic.

### (2) Results of evaluation of cell culture

FIG. 16 presents an image of the cultured-cell-comprising substance observed under a phase-contrast microscope. As illustrated in the drawing, it has been verified that the cells spread in the porous structure.

### [Example 7]

A cultured-cell-comprising substance in the present Example was produced by the technique in accordance with Example 5 except that SH-SY5Y cells (human neuroblastomas) were used in place of the NIH/3T3 cells.

### (1) Culture of SH-SY5Y cells

In an incubator, the SH-SY5Y cells were cultured in a 100 mm dish for 72 hours, using DMEM comprising 10 mass% FBS and 1 mass% antibiotic.

### (2) Results of evaluation of cell culture

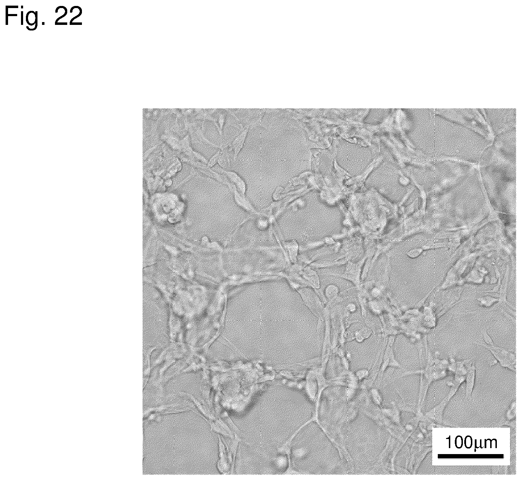

FIG. 22 presents an image of a cultured-cell-comprising substance observed under a phase-contrast microscope. As illustrated in the drawing, it has been verified that the cells spread in the cell culture carrier.

### [Example 8]

A cultured-cell-comprising substance in the present Example was produced by the technique in accordance with Example 5 except that a 96-well plate was used in place of the 35 mm dish. The porous structure and the cultured-cell-comprising substance in the 96-well plate were produced specifically using the following technique.

### (1) Formation of hydrogel

To 100 µL of a solution of acid-treated gelatin at a concentration of 1.0 (w/v)%, 1 µL of a 0.5 mg/mL laminin solution was added, and the resulting solution was mixed with stirring using a vortex mixer. Then, 1 µL of a solution of transglutaminase adjusted to 25 U/mL in PBS (-) was added, and the resulting solution was mixed with stirring using the vortex mixer. Into a 96-well plate, 50 µL of a gelatin solution comprising laminin and transglutaminase in mixture was dropped. The gelatin solution dropped into the 96-well plate was left to stand at 4°C for one day to form a hydrogel.

### (2) Production of porous structure

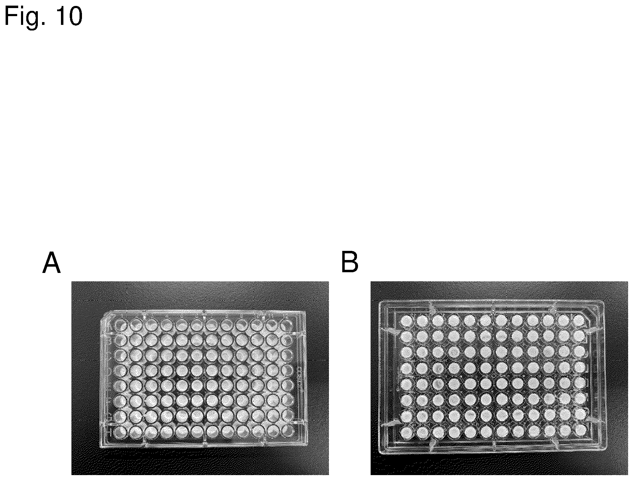

The hydrogel was frozen using a deep freezer at -150°C for 30 minutes, and then dried using a small plasma device under reduced pressure at 5 Pa or less for two hours to obtain a porous structure. FIG. 10 presents observational images of a porous structure produced in the 96-well plate. FIG. 10A is a photographed image of the front face of the 96-well plate, and FIG. 10B is a photographed image of the back face of the 96-well plate.

### (3) Production of cultured-cell-comprising substance

A cell suspension was produced by the same procedures as in Example 5. The cell concentration of the cell suspension was 1 × 10⁷ cells/mL. To the porous structure, 30 µL of the cell suspension was dropped to produce a cultured-cell-comprising substance. To the substance, 150 mL of DMEM comprising 10 mass% FBS and 1 mass% antibiotic was added, and the resulting mixture was placed in an incubator with an environment at 37°C and under 5 vol% CO₂. The cells were thus cultured.

### (4) Immunofluorescent staining of cells

The DMEM comprising 10 mass% FBS and 1 mass% antibiotic was removed, and then, PBS (-) was added to wash the cells (hereinafter referred to as "PBS washing"). The supernatant was removed, 4% paraformaldehyde (a 4% paraformaldehyde-phosphate buffer, manufactured by Fujifilm Wako Pure Chemical Corporation, hereinafter referred to as "4% PFA") was added, and the resulting solution was left to stand for 20 minutes to fix the cells. After the supernatant was removed, the PBS washing was repeated three times. The supernatant was removed, 0.1% Triton was added, and the resulting solution underwent a cell membrane permeation treatment for 10 minutes. The PBS washing was repeated three times, and the cells underwent blocking with 1% BSA for one hour. A beta-actin antibody (tradename: ab8227, from Abcam Plc) was diluted with 1% BSA to prepare a diluted liquid of a primary antibody. After the blocking, the supernatant was removed, the diluted liquid of a primary antibody was added, and the resulting liquid was incubated at 4°C overnight. The next day, the PBS washing was performed three times, a solution of a fluorescence-labeled secondary antibody diluted with a 1% BSA solution was added, and the resulting solution was incubated for one hour. The subsequent operations were performed with light blocked by an aluminum foil or the like. After the supernatant was removed, a solution of Hoechst (H3530, manufactured by Thermo Fisher) diluted with PBS was added, and the cells underwent nuclear staining for five minutes. The PBS washing was repeated twice, and 100 µL of PBS (-) was filled.

### (5) Results of Evaluation of cell culture

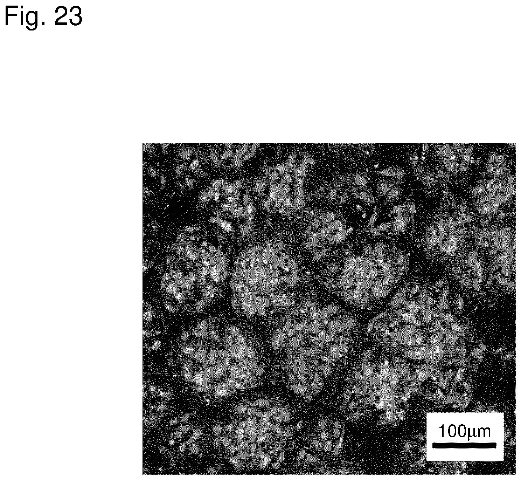

FIG. 23 presents an image of the cultured-cell-comprising substance observed under a confocal microscope. As illustrated in the drawing, it has been verified that the cells spread in the porous structure.

### [Example 9]

A cultured-cell-comprising substance in the present Example was produced by the technique in accordance with Example 5 except that HepG2 cells (human-liver-cancer-derived cell lines) were used in place of the NIH/3T3 cells. However, collagen (Collagen Type I, 10.21mg/mL, manufactured by Corning) was used as the first protein, and laminin (iMatrix-511, manufactured by Nippi) was used as the second protein.

### (1) Preparation of gel precursors

One PBS tablet (PBS (Phosphate Buffered Salts) Tablets, manufactured by Takara Bio Co., LTD.) was dissolved in pure water 10mL and filtrated through a filter having an average pore diameter of 0.2 µm (Minisart Syringe Filter 175497K, manufactured by Sartorius AG) to prepare PBS solutions 10 times more concentrated than usual. A collagenous Type I solution (Collagen Type I, 10.21mg/mL, manufactured by Corning), a reconstitution buffer solution (for Cellmatrix, manufactured by Nitta Gelatin Inc.), and the 10-fold concentration PBS were mixed with at a ratio of 8:1:1 to prepare a collagen sollution.

### (2) Formation of hydrogel

To 100 µL of the collagen solution, 1 µL of a 0.5 mg/mL laminin solution was added, and the resulting solution was mixed by pipetting. Then, 1 µL of a solution of transglutaminase adjusted to 25 U/mL in PBS (-) was added, and the resulting solution was mixed by pipetting. Into a 35 mm dish, 30 µL of the collagen solution comprising laminin and transglutaminase in mixture was dropped in dome-like form. The collagen solution dropped into the dish was put in the incubator under the condition at 37°C and under 5 vol% CO₂ for 4 hours, and then was left to stand at 4°C for one day to form a hydrogel.

### (3) Preparation of porous structures

The hydrogel was frozen in a deep freezer at -150 °C for 30 minutes, and dried using a freeze-dryer (FDS-1000, manufactured by Tokyo Rikakikai, Co., Ltd.) for 2 hours at a reduced pressure of 5Pa or less to obtain a porous structural member.

### (4) Production of cell cultures

A cell suspension was prepared in the same manner as in Example 6. The cell density of the cell suspension was 1×10⁷ /mL. A cell culture was prepared by dropping 30µL of the cell suspension into the porous structure. To the structure, DMEM 150mL containing 10% by weight FBS and 1% by weight antibiotic was added and incubated in an incubator under the condition at 37°C and under 5 vol% CO₂.

### (5) Immunofluorescent staining of cells

Immunostaining of the cells was performed in the same manner as in Example 8.

### (6) Results of cell culture evaluation

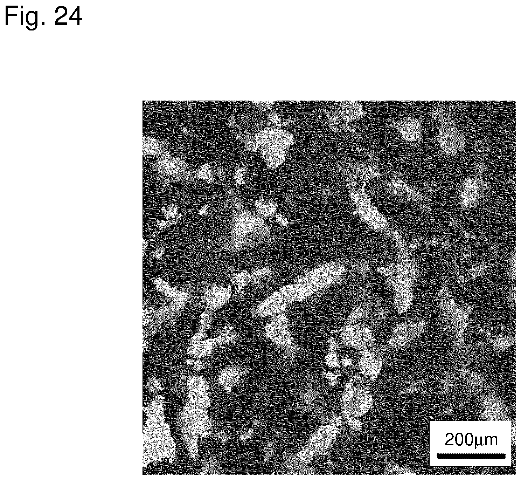

An image of the cell culture observed with a confocal microscope is shown in FIG. 24. As shown in the figure, it was confirmed that the cells were adhered and cultured inside the porous structure.

### [Example 10]

A cultured-cell-comprising substance in the present Example was produced by the technique in accordance with Example 9 except that SH-SY5Y cells (human neuroblastoma) were used in place of the HepG2 cells.

### [Example 11]

A cultured-cell-comprising substance in the present Example was produced by the technique in accordance with Example 9 except that human umbilical vein endothelial cells (Human Umbilical Vein Endothelial Cell; hereinafter referred to as "HUVEC" or "HUVEC cells") were used in place of the HepG2 cells.

### (1) Culture of HUVEC cells

HUVEC cells were incubated in a 100mm dishe for 48 hours under the condition o at 37°C and under 5 vol% CO₂ in the incubator (KM-CC17RU2, 37°C, 5 vol% CO₂, manufactured by Panasonic Corporation) using endodermal cell basic culture medium (Culture system containing EBMTM-2 Basal Medium, CC-3156, manufactured by Lonza) and EGMTM-2 SingleQuots^{™} Supplements (CC-4176, manufactured by Lonza).

### [Comparative Example 5]

MATRIGEL (Model No. 354234, manufactured by Corning) was dropped into a 35 mm dish to produce a hydrogel. The hydrogel produced was used as a cell culture carrier, in which NIH/3T3 cells were seeded to produce a cultured-cell-comprising substance. The cultured-cell-comprising substance was produced by the technique in accordance with Example 5 except that the hydrogel was used in place of the porous structure.

To the dish comprising the cultured-cell-comprising substance, 0.5 µL of an orange fluorescent dye was added to stain the cells, which were observed under a confocal microscope to evaluate whether the cells spread inside the cell culture carrier. The result has verified that the living cells were unevenly distributed on the surface of the carrier, and were hardly comprised in the carrier (data not illustrated).

As verified by the results in Example 5, it was possible that a cultured-cell-comprising substance was produced by seeding cells using, as a cell culture carrier, a porous structure formed by crosslinking the first polypeptide with the second polypeptide, and thus that cells were cultured inside the cell culture carrier. In contrast, the results in Comparative Example 4 have demonstrated that it was difficult to culture cells inside the gel (a cell culture carrier) simply by seeding the cells in MATRIGEL.

### [Comparative Example 6]

A cell suspension of NIH/3T3 cells (at a cell concentration of 1 × 10' cells/mL) and MATRIGEL were mixed in the same amount, and 30 µL of the resulting mixture was dropped into a 35 mm dish to produce a cultured-cell-comprising substance. The basic procedures in the present Comparative Example were in accordance with Comparative Example 5.

### [Comparative Example 7]

A cell suspension of NIH/3T3 cells (at a cell concentration of 1 × 10⁷ cells/mL) and MATRIGEL were mixed in the same amount, and 50 µL of the resulting mixture was dropped into a 96-well plate to produce a cultured-cell-comprising substance. The basic procedures in the present Comparative Example were in accordance with Example 8. However, differently from Example 7, no porous structure was formed.

### [Evaluation of Survival Rate]

In Examples 5 to 11 and Comparative Examples 5 to 7, the cell survival rate of the cultured-cell-comprising substance was evaluated.

### (1) Preparation of survival rate evaluating solution

To a DMEM medium comprising 10 mass% FBS and 1 mass% antibiotic, PI (P1304MP, manufactured by Thermo Fisher Scientific Inc.) and Hoechst (H3570, manufactured by Thermo Fisher Scientific Inc.) were added at a dilution ratio of 2000 times to produce a survival rate evaluating solution.

### (2) Observation of cells

After the culture, 2 mL of the medium in the 35 mm dish was replaced with 2 mL of the survival rate evaluating solution, and the cells were again cultured for two hours in an incubator with an environment at 37°C and under 5 vol% CO₂. After the culture, the cultured-cell-comprising substance was observed under a confocal microscope to obtain three-dimensional images of PI and Hoechst observed under fluorescence. Table 1 comprises the number of days for culture, in which days the survival rate was evaluated in Examples 5 to 11 and Comparative Examples 5 to 7.

### (3) Calculation of survival rate

On the basis of the three-dimensional images, the cells stained with PI were regarded as dead cells, the cells stained with Hoechst were regarded as whole cells, and the survival rate (%) was calculated in accordance with the following: (the number of whole cells - the number of dead cells) / (the number of whole cells) × 100. The cell survival rate of 80% or more was rated Good, and less than 80% was rated Poor. The results are given in Table 1.

### [Evaluation of Toxicity]

In the case of Example 8 and Comparative Example 7, the toxicity of the cells in the cultured-cell-comprising substance was evaluated besides the cell survival rate. Different Examples and Comparative Examples were evaluated with different samples produced by the same method.

### (1) Preparation of reagent

A 30% hydrogen peroxide solution and pure water were mixed to produce a 1% hydrogen peroxide solution, which was filtrated through a filter (Minisart Syringe Filter 175497K, manufactured by Sartorius AG) having an average pore diameter of 0.2 µm.

### (2) Addition of reagent

The medium was collected from the well in Example 8 and Comparative Example 7, and washed with PBS three times. After the washing, 50 µL of the 1% hydrogen peroxide solution was added to the well, and left to stand at room temperature for 10 minutes. After thus standing for 10 minutes, the hydrogen peroxide solution was collected, and washed with PBS (-) three times. After the washing, 150 µL of the survival rate solution was added, and the cells were cultured for two hours in an incubator with an environment at 37°C and under 5 vol% CO₂. After culturing for two hours, the cultured-cell-comprising substance was observed under a confocal microscope to obtain three-dimensional images of PI and Hoechst observed under fluorescence.

### (3) Calculation of survival rate

On the basis of the three-dimensional images, the cells stained with PI were regarded as dead cells, the cells stained with Hoechst were regarded as whole cells, and the survival rate (%) was calculated in accordance with the following: (the number of whole cells - the number of dead cells) / (the number of whole cells) × 100. The cell survival rate calculated was rated "Good" if the survival rate in the Evaluation of Toxicity is lower than that in the Evaluation of Survival Rate, and rated "Poor" if the survival rate in the Evaluation of Toxicity is equal to or higher than that in the Evaluation of Survival Rate. The results are given in Table 1.

### [Results of Evaluation of Survival Rate and Evaluation of Toxicity]

Table 1 summarizes the results of Examples 5 to 11 and Comparative Examples 5 to 7 of the cultured-cell-comprising substance. It has been verified that seeding and culturing cells in the porous structures in Examples 5 to 7 to produce cultured-cell-comprising substances enabled the cells to be cultured in the porous structures. It has been demonstrated that, because a porous structure according to the present invention has a plurality of pores, cells can infiltrate into the porous structure and that, because the porous structure is produced with a polypeptide adhesive to cells, many cells can be seeded and cultured in the porous structure. It has also been verified that various species of cells can be seeded in the porous structure. According to the results of Comparative Example 5, simply seeding cells in MATRIGEL (a cell culture hydrogel carrier for culture) made it difficult to culture the cells inside the carrier, and it was not possible to evaluate the survival rate of the cells. It has been demonstrated that such a gel having no porous architecture as MATRIGEL used in Comparative Example 5 did not enable cells to infiltrate into the MATRIGEL, and thus did not allow such a three-dimensional culture as in Examples 5 to 7. It has also been verified that, in Comparative Example 6, the cells were embedded in MATRIGEL, and a cell culture inside the carrier was attempted, but the survival rate was decreased. It has been demonstrated that, as in Comparative Example 6, a three-dimensional culture in a gel having no porous architecture allowed neither oxygen nor nutrients to reach the inside of the gel, and thus failed to prevent the cells from dying. In Example 8, the evaluation of toxicity of the cultured-cell-comprising substance was possible, but in Comparative Example 7, the permeability of the gel was poor, the cells were not stained, and thus, neither the evaluation of survival rate nor the evaluation of toxicity was possible. As described above, it has been demonstrated that a porous structure according to the present invention has a high solution-permeability, and not only prevents the death of cells inside a cell culture carrier, but also makes it easy to observe the form of cells by staining, and to evaluate the effect of a drug or the like on cells.

**[Table 1]**

| Porous Structure | Example 5 | Example 6 | Example 7 | Example 8 | Example 9 | Example 10 | Example 11 | Comparative Example 5 | Comparative Example 6 | Comparative Example 7 |
|---|---|---|---|---|---|---|---|---|---|---|
| First Polypeptide | Gelatin Type A | Gelatin Type A | Gelatin Type A | Gelatin Type A | Collagen | Collagen | Collagen | MATRIGEL | MATRIGEL (cell suspension) | MATRIGEL (cell suspension) |
| Second Polypeptide | Laminin | Laminin | Laminin | Laminin | Laminin | Laminin | Laminin | none | none | none |
| Species of Cell | NIH/3T3 Cell | HepG2 Cell | SH-SY5Y Cell | NIH/3T3 Cell | HepG2 Cell | SH-SY5Y Cell | HUVEC cell | NIH/3T3 Cell | NIH/3T3 Cell | NIH/3T3 Cell |
| Container | 35 mm Dish | 35 mm Dish | 35 mm Dish | 96-well Plate | 35 mm Dish | 35 mm Dish | 35 mm Dish | 35 mm Dish | 35 mm Dish | 96-well Plate |
| Number of Days for Culture | 6 days | 4 days | 4 days | 1 day | 6 days | 4 days | 4 days | 4 days | 4 days | 1 day |
| Evaluation of Survival Rate | Good | Good | Good | Good | Good | Good | Good | unmeasurable | Poor | unmeasurable |
| Evaluation of Toxicity | - | - | - | Good | - | - | - | - | - | unmeasurable |

| | | | | | | | | | | |
|---|---|---|---|---|---|---|---|---|---|---|
| -: not evaluated | | | | | | | | | | |

The present invention encompasses the following aspects.
[1] A porous structure comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the porous structure.
[2] The porous structure according to [1], wherein at least one of the first polypeptide and the second polypeptide has at least one glutamine residue, and wherein the other has at least one lysine residue.
[3] The porous structure according to [1] or [2], wherein the first polypeptide comprises a protein selected from the group consisting of collagen, gelatin, fibrin, and elastin.
[4] The porous structure according to any one of [1] to [3], wherein the second polypeptide comprises a protein selected from the group consisting of laminin, fibronectin, thrombin, vimentin, heparin, and vitronectin.
[5] The porous structure according to any one of [1] to [4], wherein the average pore size of the porous structure is 5 to 1000 µm.
[6] The porous structure according to any one of [1] to [5], further comprising at least one component selected from the group consisting of a cell adhesion factor, cell growth factor, and cell differentiation control factor.
[7] A cultured-cell-comprising substance comprising the porous structure according to any one of [1] to [6] and a cell.
[8] The cultured-cell-comprising substance according to [7], wherein the cell comprises at least one kind of cell selected from the group consisting of a liver cell; endothelial cells such as a stellate cell, Kupffer cell, vascular endothelial cell, sinusoidal endothelial cell, and corneal endothelial cell; epidermal cells such as a fibroblast, osteoblast, osteoclast, periodontium-derived cell, and epidermal keratinial cell; epithelial cells such as a tracheal epithelial cell, gastrointestinal epithelial cell, uterocervical epithelial cell, and corneal epithelial cell; muscle cells such as a mammary glandular cell, pericyte, smooth muscle cell, and myocardial cell; a renal cell; a pancreas islet cell; a chondrocyte; an osteocyte; a neurocyte; a hybridoma; a hemocyte-derived cell; and an ovarian cell.
[9] A method for producing a porous structure, comprising:
   preparing a liquid mixture comprising at least a first polypeptide and a second polypeptide;
   adding transglutaminase to the liquid mixture and allowing the resulting mixture to react to form a gel; and
   forming the porous structure from the gel.
[10] A method for producing a porous structure according to [9], wherein the transglutaminase is derived from a microorganism.
[11] A method for culturing a cell, comprising:
   seeding a cell in the porous structure according to any one of [1] to [6]; and
   culturing the cell in the porous structure.
[12] A method for producing a cultured-cell-comprising substance, comprising:
   seeding a cell in the porous structure according to any one of [1] to [6]; and
   culturing the cell in the porous structure.
[13] A hydrogel structure comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the structure.
[14] A method for producing a hydrogel structure, comprising:
   preparing a liquid mixture comprising at least a first polypeptide and a second polypeptide; and
   adding transglutaminase to the liquid mixture and allowing the resulting mixture to react to form a gel.

## Claims

1. A porous structure comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the porous structure.

2. The porous structure according to claim 1, wherein at least one of the first polypeptide and the second polypeptide has at least one glutamine residue, and wherein the other has at least one lysine residue.

3. The porous structure according to claim 1 or 2, wherein the first polypeptide comprises a protein selected from the group consisting of collagen, gelatin, fibrin, and elastin.

4. The porous structure according to any one of claims 1 to 3, wherein the second polypeptide comprises a protein selected from the group consisting of laminin, fibronectin, thrombin, vimentin, heparin, and vitronectin.

5. The porous structure according to any one of claims 1 to 4, wherein the average pore size of the porous structure is 5 to 1000 µm.

6. The porous structure according to any one of claims 1 to 5, further comprising at least one component selected from the group consisting of a cell adhesion factor, cell growth factor, and cell differentiation control factor.

7. A cultured-cell-comprising substance comprising the porous structure according to any one of claims 1 to 6 and a cell.

8. The cultured-cell-comprising substance according to claim 7, wherein the cell comprises at least one kind of cell selected from the group consisting of: a liver cell; endothelial cells such as a stellate cell, Kupffer cell, vascular endothelial cell, sinusoidal endothelial cell, and corneal endothelial cell; epidermal cells such as a fibroblast, osteoblast, osteoclast, periodontium-derived cell, and epidermal keratinial cell; epithelial cells such as a tracheal epithelial cell, gastrointestinal epithelial cell, uterocervical epithelial cell, and corneal epithelial cell; muscle cells such as a mammary glandular cell, pericyte, smooth muscle cell, and myocardial cell; a renal cell; a pancreas islet cell; a chondrocyte; an osteocyte; a neurocyte; a hybridoma; a hemocyte-derived cell; and an ovarian cell.

9. A method for producing a porous structure, comprising:
preparing a liquid mixture comprising at least a first polypeptide and a second polypeptide;
adding transglutaminase to the liquid mixture and allowing the resulting mixture to react to form a gel; and
forming the porous structure from the gel.

10. The method for producing a porous structure according to claim 9, wherein the transglutaminase is derived from a microorganism.

11. A method for culturing a cell, comprising:
seeding a cell in the porous structure according to any one of claims 1 to 6; and
culturing the cell in the porous structure.

12. A method for producing a cultured-cell-comprising substance, comprising:
seeding a cell in the porous structure according to any one of claims 1 to 6; and
culturing the cell in the porous structure.

13. A hydrogel structure comprising at least a first polypeptide and a second polypeptide, wherein at least a part of the first polypeptide and at least a part of the second polypeptide are crosslinked with each other via an isopeptide bond, and wherein the first polypeptide and the second polypeptide are dispersed overall in the structure.

14. A method for producing a hydrogel structure, comprising:
preparing a liquid mixture comprising at least a first polypeptide and a second polypeptide; and
adding transglutaminase to the liquid mixture and allowing the resulting mixture to react to form a gel.
